# EUROPÄISCHE PATENTSCHRIFT

(11) **EP 1 555 258 B1**
(45) Veröffentlichungstag und Bekanntmachung des Hinweises auf die Patenterteilung: **05.11.2008**
(21) Anmeldenummer: 04028815.1
(22) Anmeldetag: 06.12.2004
(51) Int. Cl.: C07C 263/10, C07C 265/14

(54) **Verfahren zur Herstellung von Diisocyanaten und/oder Triisocyanaten**
Preparation of di- and/or triisocyanates
Procédé pour la préparation de diisocyanates et/ou triisocyanates

(30) Priorität: 18.12.2003 DE 10359627
(43) Veröffentlichungstag der Anmeldung: 20.07.2005
(73) Patentinhaber: Bayer MaterialScience AG, 51368 Leverkusen (DE)
(72) Erfinder: Meyn, Jürgen Dr., 41542 Dormagen (DE); Stutz, Herbert Dr., 41541 Dormagen (DE)

(56) Entgegenhaltungen:
- EP-A- 1 319 655
- FR-A- 2 325 637
- US-A- 4 847 408
- US-A- 5 633 396
- US-B1- 6 225 497

## Beschreibung

Die vorliegende Erfindung betrifft ein Verfahren zur Herstellung von Diisocyanaten und / oder Triisocyanaten durch Phosgenierung der entsprechenden Diamine und / oder Triamine in der Gasphase.

Die Herstellung von Isocyanaten durch Umsetzung von Aminen mit Phosgen in der Gasphase ist seit langem bekannt (vgl. Siefken, Annalen 562, 108 (1949). Gasphasenreaktionen können auf unterschiedlichste Art durchgeführt werden. Zur Mischung der Edukte werden Düsen, Brenner oder Mischrohre eingesetzt. Für die Gasphasenphosgenierung von Diisocyanaten ist der Einsatz von Düsen ganz allgemein beschrieben. Dabei handelt es sich, wie z.B. in EP-A1-0593334 beschrieben, um Glattstrahldüsen bzw. konzentrische Einleitrohre. Dabei wird üblicherweise eines der Edukte durch eine zentrisch angeordnete Düse in den Strom des zweiten Edukts, das durch den Ringraum um das Düsenrohr mit geringer Geschwindigkeit strömt, eingedüst. Das schneller strömende Edukt saugt dabei das langsam strömende Edukt an und es kommt zur Vermischung. Nach einer vom Düsendurchmesser und dem Unterschied in den Strömungsgeschwindigkeiten der Edukte abhängigen Zeit bzw. Weg wird dann eine vollständige Vermischung der Edukte erreicht. Der Vermischung überlagert ist die chemische Reaktion. Die Gasphasenphosgenierung von Aminen ist eine Reaktion, deren Geschwindigkeit von der Vermischung der Edukte bestimmt wird. Da die entstehenden Isocyanate mit den Aminen Folgereaktionen eingehen können, ist zur Erzielung einer hohen Selektivität zum gewünschten Diisocyanat eine schnelle Vermischung und ein Phosgenüberschuss notwendig. Aufgrund von Rückvermischungsvorgängen kommt es zur Reaktion des Diisocyanats mit unreagiertem Diamin aus dem Eduktstrom unter Entstehung von festen Ablagerungen. Dadurch kommt es zur Verschmutzung des Reaktors unterhalb der Mischzone und zu Verstopfungen des Reaktors.

Bei einer Vergrößerung der Reaktors, der häufig als Rohrreaktor ausgebildet ist, wird auch eine Vergrößerung der Mischdüse, die häufig als Glattstrahldüse ausgebildet ist, notwendig. Mit der Vergrößerung des Durchmessers der Glattstrahldüse wird aber auch die Geschwindigkeit der Vermischung des Zentralstrahls durch den größeren erforderlichen Diffusionsweg verringert und die Gefahr von Rückvermischung erhöht, was wiederum zur Entstehung polymerer Verunreinigungen und damit fester Anbackungen im Reaktor führt.

In der GB-PS 1165831 wird die Reaktion in einem mit einem mechanischen Rührer ausgestatteten Rohrreaktor durchgeführt. Der Reaktor ähnelt einem Dünnschichtverdampfer, bei dem der Rührer die Gase mischt und gleichzeitig die beheizten Wände des Rohrreaktors bestreicht, um so einen Aufbau von polymerem Material an der Rohrwand zu verhindern. Die Verwendung eines schnelllaufenden Rührers beim Umgang mit ca. 300°C heißem Phosgen erfordert jedoch hohen sicherheitstechnischen Aufwand, um den Reaktor abzudichten und den Rührer in dem hochkorrosiven Medium zu lagern.

FR 2 325 637 beschreibt die Phosgenierung von Aminen in der Flüssigphase, wobei mehrer parallel geführte Teilströme aus Monochlorbenzol-Phosgen- bzw. Monochlorbenzol-Amin-Mischungen miteinander umgesetzt werden. Hinweise auf die Querschnittsflächen der Teilströme bzw. eine Übertagbarkeit auf die Phosgenierung in der Gasphase werden nicht gegeben.

US 5 633 396 und EP 1 319 655 betreffen beide die Gasphasenphosgenierung, wobei in letzterer Anmeldung Bezug auf den Einfluss durch Reaktorgeometrie und Größenverhältnisse genommen wird. Hinweise auf die Einsetzbarkeit einer Ringspaltdüse oder gar deren Vorzüge wird jedoch nicht gegeben.

Aufgabe der vorliegenden Erfindung ist daher, ein Verfahren zur Herstellung von Diisocyanaten und/oder Triisocyanaten in der Gasphase bereitzustellen, bei dem die Edukte Diamin und Phosgen schneller und besser in einem Reaktor ohne bewegte Einbauten vermischt werden können und bei dem die Entstehung polymerer Verunreinigungen und von Anbackungen im Reaktor vermieden werden können.

Es wurde nun gefunden, dass es möglich ist, (cyclo)aliphatische oder aromatische Diisocyanate und/oder Triisocyanate durch Gasphasenphosgenierung der entsprechenden Diamine und/oder Triamine unter Eliminierung der genannten Nachteile des Standes der Technik herzustellen, wenn der eine Eduktstrom über einen Ringspalt, der konzentrisch im Strom des anderen Eduktes angebracht ist, mit hoher Geschwindigkeit eingemischt wird. Dadurch werden der Diffusionsweg für die Vermischung klein und die Mischzeiten sehr kurz. Die Reaktion kann dann mit hoher Selektivität zum gewünschten Diisocyanat ablaufen. Die Entstehung von polymeren Verunreinigungen und Anbackungen werden dadurch verringert.

Gegenstand der Erfindung ist ein Verfahren zur Herstellung von Diisocyanaten und Triisocyanaten der allgemeinen Formel (I)

R(NCO)ₙ (I),

in welcher
- R: für einen (cyclo)aliphatischen oder aromatischen Kohlenwasserstoffrest mit bis zu 15 Kohlenstoffatomen, vorzugsweise 4 bis 13 Kohlenstoffatomen, steht, mit der Maßgabe, dass zwischen zwei NCO-Gruppen mindestens 2 Kohlenstoffatome angeordnet sind und
- n: für die Zahl 2 oder 3 steht,
durch Phosgenierung der entsprechenden Diamine und/oder Triamine der allgemeinen Formel (II)

R(NH₂)ₙ (II),

in welcher
- R: für einen (cyclo)aliphatischen oder aromatischen Kohlenwasserstoffrest mit bis zu 15, vorzugsweise 4 bis 13 Kohlenstoffatomen steht, mit der Maßgabe, dass zwischen zwei Aminogruppen mindestens zwei Kohlenstoffatome angeordnet sind und
- n: für die Zahl 2 oder 3 steht,
in der Gasphase in einem Rohrreaktor, der ein zentrisch in Richtung der Rotationsachse der Rohrreaktors ausgedehntes doppelwandiges Leitrohr aufweist, wobei zwischen der inneren und der äußeren Wand des doppelwandigen Leitrohrs ein konzentrischer Ringspalt ausgebildet ist, und wobei das Verhältnis der Querschnittsfläche des Rohrreaktors, die von der inneren Wand des doppelwandigen Leitrohrs begrenzt wird, zu der Querschnittsfläche des Rohrreaktors, die von der Wand des Rohrreaktors und der äußeren Wand des doppelwandigen Leitrohrs begrenzt wird, 1:0,5 bis 1:4, bevorzugt 1:1 bis 1:3, beträgt,
bei dem die dampfförmigen Diamine und / oder Triamine und Phosgen getrennt voneinander auf Temperaturen von 200°C bis 600°C erhitzt werden,
und die dampfförmigen Diamine und / oder Triamine dem Rohrreaktor über den konzentrischen Ringspalt mit einer mittleren Strömungsgeschwindigkeit von 20-150 m/s, vorzugsweise 40-100m/s, zugeführt werden und Phosgen dem Rohrreaktor auf der verbleibenden Querschnittsflächen des Rohrreaktors mit einer mittleren Strömungsgeschwindigkeit von mindestens 1 m/s, vorzugsweise 5-15 m/s zugeführt wird.

Die dampfförmigen Diamine können auch gegebenenfalls verdünnt mit einem Inertgas oder mit den Dämpfen eines inerten Lösungsmittels dem Rohrreaktor zugeführt werden. Geeignete Inertgase sind beispielsweise Stickstoff oder Edelgase wie Helium oder Argon. Vorzugsweise wird Stickstoff eingesetzt. Geeignete Lösungsmittel sind beispielsweise Chlorbenzol, o-Dichlorbenzol, Toluol, Xylol, Chlortoluol, Chlornaphthalin, Decahydronaphthalin. Vorzugsweise wird Chlorbenzol eingesetzt.

Die Vermischung der beiden gasförmigen Edukte findet nach dem erfindungsgemäßen Verfahren an den ringförmigen Trennflächen der Eduktstrahlen Diamin und Phosgen statt.

Ausgangsmaterialien für das erfindungsgemäße Verfahren sind Diamine und/oder Triamine der allgemeinen Formel (In

R(NH₂)ₙ (II),

in welcher
- R: für einen (cyclo)aliphatischen oder aromatischen Kohlenwasserstoffrest mit bis zu 15, vorzugsweise 4 bis 13 Kohlenstoffatomen steht, mit der Maßgabe, dass zwischen zwei Aminogruppen mindestens zwei Kohlenstoffatome angeordnet sind und
- n: für die Zahl 2 oder 3 steht.

Typische Beispiele geeigneter aliphatischer Diamine sind in EP-A1-0289840 in Spalte 3, Zeilen 19 bis 26 genannt. Beispiele geeigneter aliphatischer Triamine sind z.B. in EP-A-749 958 in Spalte 3, Zeilen 18 bis 22 und Zeilen 28 bis 31 genannt. Besonders geeignet sind 1,4-Diaminobutan, 1,3-Diaminopentan, 1,6-Diaminohexan (HDA), 1,11-Diaminoundecan, 1,4-Diaminocyclohexan, 1-Amino-3,3,5-trimethyl-5-aminomethylcyclohexan (Isophorondiamin, IPDA), 2,3-,2,4- und 2,6-Diamino-1-methylcyclohexan sowie deren Gemische, 1,3,5-Triisopropyl-2,4-diaminocyclohexan, 2,4- und 2,6-Diamino-1-isopropylcyclohexan oder deren Gemische und Bis-(p-aminocyclohexyl)-methan.

Bevorzugt sind Isophorondiamin (IPDA), Hexamethylendiamin (HDA) und Bis(p-aminocyclohexyl)methan.

Typische Beispiele geeigneter aromatischer Diamine sind die reinen Isomeren oder die Isomerengemische des Diaminobenzols, des Diaminotoluols, des Diaminodimethylbenzols, des Diaminonaphthalins sowie des Diaminodiphenylmethans, bevorzugt sind 2,4/2,6-Toluylendiamin-Gemische der Isomerenverhältnisse 80/20 und 65/35 oder das reine 2,4-Toluylendiamin-Isomere.

Als Triamin wird vorzugsweise 1 ,8-Diamino-4-(aminomethyl)octan, Triaminononan eingesetzt.

Die Ausgangsamine werden vor der Durchführung des erfindungsgemäßen Verfahrens verdampft und auf 200°C bis 600°C, vorzugsweise 300°C bis 500°C erhitzt und gegebenenfalls verdünnt mit einem Inertgas oder mit den Dämpfen eines inerten Lösungsmittels dem Reaktor zugeführt.

Das bei der Phosgenierung verwendete Phosgen wird vor Durchführung des erfindungsgemäßen Verfahrens ebenfalls auf eine Temperatur innerhalb des Bereichs von 200°C bis 600°C, vorzugsweise 300°C bis 500°C erhitzt.

Zur Durchführung der erfindungsgemäßen Umsetzung werden der vorerhitzte Strom enthaltend Diund / oder Triamine oder Gemische von Di- und / oder Triaminen und der vorerhitzte Strom enthaltend Phosgen kontinuierlich in den Rohrreaktor geleitet.

Die Rohrreaktoren bestehen im allgemeinen aus Stahl, Glas, legiertem oder emaillierten Stahl und weisen eine Länge auf, die ausreichend ist, um unter den Verfahrensbedingungen eine vollständige Umsetzung des Diamins mit dem Phosgen zu ermöglichen. Der Phosgenstrom wird im allgemeinen an einem Ende des Rohrreaktors zugeführt. In diesen Phosgenstrom wird über einen radialsymmetrisch angebrachten konzentrischen Ringspalt das Amin mit hoher Geschwindigkeit eingemischt. Das Phosgen wird dem Rohrreaktor dabei sowohl über die Querschnittsfläche, die von der inneren Wand des doppelwandigen Leitrohrs begrenzt wird als auch über die Querschnittsfläche, die von der Wand des Rohrreaktors und der äußeren Wand des doppelwandigen Leitrohrs begrenzt wird, zugeführt.

Die Mischzone wird bevorzugt bei einer Temperatur innerhalb des Bereiches von 200°C und 600°C, vorzugsweise 300°C und 500°C gehalten, wobei diese Temperatur gegebenenfalls durch Beheizung des Rohrreaktors aufrecht gehalten werden kann.

Bei der Durchführung des erfindungsgemäßen Verfahrens liegt der Druck in den Zuleitungen zum Rohrreaktor bevorzugt bei 200 mbar bis 4000 mbar und am Ausgang aus dem Rohrreaktor bei 150 mbar bis 2000 mbar. Durch die Aufrechterhaltung eines geeigneten Differenzdruckes wird eine Strömungsgeschwindigkeit des Phosgenstroms am Eintritt in den Rohrreaktor von mindestens 1 m/s, bevorzugt 2 m/s bis 60 m/s, besonders bevorzugt 3 bis 20 m/s, ganz besonders bevorzugt 5 bis 15 m/s eingestellt.

Die Einmischung des Amins erfolgt über einen konzentrischen Ringspalt mit einer Geschwindigkeit von 20-150 m/s, vorzugsweise 40-100m/s. Die Vermischung der beiden gasförmigen Edukte Diamin und Phosgen findet an den ringförmigen Trennflächen der Eduktstrahlen statt.

Unter diesen Reaktionsbedingungen herrschen innerhalb des Reaktionsraumes im allgemeinen turbulente Strömungsverhältnisse vor.

Die Erfindung wird nachfolgend anhand der Figur erläutert.

Die Figur zeigt einen Rohrreaktor 1 der für den Einsatz in dem erfindungsgemäßen Verfahren geeignet ist. Der Rohrreaktor 1 enthält eine zylindrische Wandung 2, die den Reaktionsraum 9 umspannt und einen Deckel 3, der den zylindrischen Reaktionsraum an einem Ende der zylindrischen Wandung 2 nach außen hin abschließt. Auf der dem Deckel 3 gegenüberliegenden Seite ist der Rohrreaktor 1 offen. In dem Deckel 3 ist zentrisch, das heißt rotationssymmetrisch zur Rotationsachse 8 der zylindrischen Wandung 2, eine Öffnung angeordnet, die von einem auf beiden Seiten des Deckels 3 überstehenden zylindrischen Rohrstück 4 ausgefüllt ist. An der in den Reaktionsraum 9 hineinragenden Seite mündet das Rohrstück 4 über Verbindungsleitungen 5 in ein doppelwandiges Leitrohr 6, das zentrisch, das heißt rotationssymmetrisch zur Rotationsachse 8 der zylindrischen Wandung 2, im Reaktionsraum 9 angeordnet ist. Der Rohrreaktor 1 weist weiterhin auf Höhe des Rohrstücks 4 einen auf der zylindrischen Wandung 2 angeordneten Einlassstutzen 7 auf.

Der Diamine und / oder Triamine enthaltende Strom A strömt durch das Rohrstück 4, die Verbindungsleitungen 5 und das doppelwandige Leitrohr 6 und tritt schließlich aus dem doppelwandigen Leitrohr in Form eines Ringstrahls aus. Der Phosgen enthaltende Strom B strömt in etwa auf Höhe des Rohrstücks 4 durch den Einlassstutzen 7 direkt in den Raum zwischen der zylindrischen Wandung 2 und dem Rohrstück 4 ein und umströmt das Rohrstück 4, die Verbindungsleitungen 5 sowie das doppelwandige Leitrohr 6. Dabei wird das doppelwandige Leitrohr 6 sowohl durch die freie Querschnittsfläche, die von der inneren Wand des doppelwandigen Leitrohrs begrenzt wird als auch durch die freie Querschnittsfläche, die von der zylindrischen Wandung 2 des Rohrreaktors und der äußeren Wand des doppelwandigen Leitrohrs begrenzt wird, umströmt. Die Strömungswege der Edukte A und B sind dabei durch die stromlinienartigen Pfeile in der Figur angedeutet. Der die Di- und / oder Triamine enthaltende Strom A tritt aus dem doppelwandigen Leitrohr 6 in Form eines ringförmigen Freistrahls aus und vermischt sich dann bei im allgemeinen turbulenter Strömung mit dem Phosgen enthaltenden Strom B, wobei sich die entsprechenden Di- und / oder Triisocyanate bilden.

### Beispiel

### Beispiel 1 (erfindungsgemäßes Beispiel):

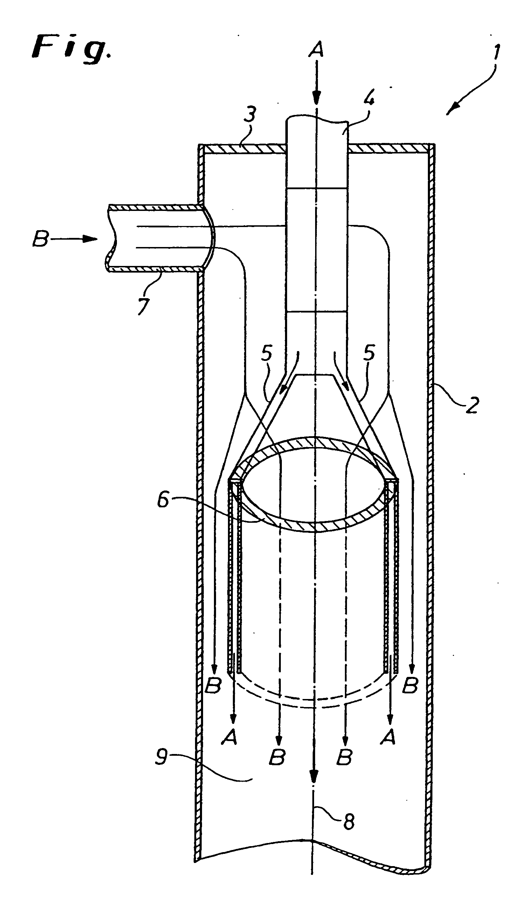

Einem Rohrreaktor gemäß Fig.1 mit nachgeschalteter Isocyanatkondensationsstufe und dieser nachfolgenden Isocyanataufarbeitung werden als Eduktstrom A ein Isophorondiamin-Inertgas-Gemisch und als Eduktstrom B Phosgen kontinuierlich zugeleitet. Die Temperaturen der beiden Eduktströme betragen 300°C. Der Druck in dem Rohrreaktor liegt geringfügig oberhalb des Atmosphärendruckes bei 1400 mbar.

Die Geschwindigkeit der Komponente A in dem doppelwandigen Leitrohr 6 beträgt ca. 60 m/s, die der Komponente B vor der Vermischung ca. 7 m/s. Dabei beträgt das Verhältnis der Querschnittsfläche des Rohrreaktors 1, die von der inneren Wand des doppelwandigen Leitrohrs 6 begrenzt wird, zu der Querschnittsfläche des Rohrreaktors, die von der zylindrischen Wandung 2 des Rohrreaktors und der äußeren Wand des doppelwandigen Leitrohrs begrenzt wird, 1:1.

Die Geschwindigkeit der Reaktionsgemisches am Reaktorausgang beträgt ca. 17 m/s.

Das Reaktionsprodukt Isophorondiisocyanat (IPDI) wird nach Verlassen des Reaktors kondensiert, von überschüssigem Phosgen und dem Nebenprodukt Chlorwasserstoff getrennt und anschließend einer Reinigung zugeführt. An vier stromabwärts des doppelwandigen Leitrohrs 6 gelegenen Temperaturmessstellen wird die Temperatur auf der zylindrischen Außenwand 2 des Rohrreaktors 1 mit Hilfe von Thermoelementen gemessen. Die Maximaltemperatur wird an der zweiten Temperaturmessstelle, die etwa zwei Durchmesser der zylindrischen Wandung 2 weit entfernt stromabwärts von der Mischstelle liegt, erreicht. Die Ausbeute an IPDI bezogen auf das eingesetzte IPDA beträgt 98,8 % der Theorie.

### Beispiel 2 (Vergleichsbeispiel):

Beispiel 1 wird unter den gleichen Bedingungen wiederholt, wobei anstelle doppelwandigen Leitrohrs eine Glattstrahldüse eingesetzt wird. Dabei gleichen die durchströmten Querschnittsflächen für das Isophorondiamin-Inertgas-Gemisch und Phosgen am Austritt aus der Düse den durchströmten Querschnittsflächen im Rohrreaktor nach Beispiel 1.

Dabei zeigt sich, dass bei dem Einsatz der konventionellen Glattstrahldüse bei vergleichbaren Geschwindigkeiten der Komponenten an der Mischstelle die Maximaltemperatur im Rohrreaktor erst deutlich später, nämlich erst etwa fünf Durchmesser der zylindrischen Wandung 2 weit entfernt stromabwärts von der Mischstelle, erreicht wird. Die Ausbeute an IPDI bezogen auf das eingesetzte IPDA beträgt 98,5 % der Theorie.

Zusätzlich zeigt sich, dass durch die bessere und schnellere Vermischung beim erfindungsgemäßen Einsatz des Rohrreaktors mit doppelwandigem Leitrohr die Bildung von polymeren Nebenprodukten, die sich an der Wand des Rohrreaktors niederschlagen, reduziert wird. Das führt zu einer längeren Standzeit des Reaktors.

## Patentansprüche

1. Verfahren zur Herstellung von Diisocyanaten und Triisocyanaten der allgemeinen Formel (I)
R(NCO)ₙ (I),
in welcher
R für einen (cyclo)aliphatischen oder aromatischen Kohlenwasserstoffrest mit bis zu 15 Kohlenstoffatomen, vorzugsweise 4 bis 13 Kohlenstoffatomen, steht, mit der Maßgabe, dass zwischen zwei NCO-Gruppen mindestens 2 Kohlenstoffatome angeordnet sind und
n für die Zahl 2 oder 3 steht,
durch Phosgenierung der entsprechenden Diamine und/oder Triamine der allgemeinen Formel (II)
R(NH₂)ₙ (II),
in welcher
R für einen (cyclo)aliphatischen oder aromatischen Kohlenwasserstoffrest mit bis zu 15, vorzugsweise 4 bis 13 Kohlenstoffatomen steht, mit der Maßgabe, dass zwischen zwei Aminogruppen mindestens zwei Kohlenstoffatome angeordnet sind und
n für die Zahl 2 oder 3 steht,
in der Gasphase in einem Rohrreaktor, der ein zentrisch in Richtung der Rotationsachse der Rohrreaktors ausgedehntes doppelwandiges Leitrohr aufweist, wobei zwischen der inneren und der äußeren Wand des doppelwandigen Leitrohrs ein konzentrischer Ringspalt ausgebildet ist, und wobei das Verhältnis der Querschnittsfläche des Rohrreaktors, die von der inneren Wand des doppelwandigen Leitrohrs begrenzt wird, zu der Querschnittsfläche des Rohrreaktors, die von der Wand des Rohrreaktors und der äußeren Wand des doppelwandigen Leitrohrs begrenzt wird, 1:0,5 bis 1:4 beträgt,
bei dem die dampfförmigen Diamine und / oder Triamine und Phosgen getrennt voneinander auf Temperaturen von 200°C bis 600°C erhitzt werden,
und die dampfförmigen Diamine und / oder Triamine dem Rohrreaktor über den konzentrischen Ringspalt mit einer mittleren Strömungsgeschwindigkeit von 20-150 m/s zugeführt werden und Phosgen dem Rohrreaktor auf der verbleibenden Querschnittsflächen des Rohrreaktors mit einer mittleren Strömungsgeschwindigkeit von mindestens 1 m/s zugeführt wird.

2. Verfahren nach Anspruch 1, bei dem die mittlere Strömungsgeschwindigkeit der dampfförmigen Diamine und / oder Triamine 40 bis 100 m/ s beträgt.

3. Verfahren nach einem der Ansprüche 1 oder 2, bei dem die mittlere Strömungsgeschwindigkeit des Phosgens 5 bis 15 m/s beträgt.

4. Verfahren nach einem der Ansprüche 1 bis 3, bei dem als Diamine Isophorondiamin, Hexamethylendiamin oder Bis(p-aminocyclohexyl)methan eingesetzt werden.

5. Verfahren nach einem der Ansprüche 1 bis 3, bei dem als Triamine 1,8-Diamino-4-(aminomethyl)octan oder Triaminononan eingesetzt werden.

6. Verfahren nach einem der Ansprüche 1 bis 4, bei dem die Phosgenierung in einem Rohrreaktor durchgeführt wird, bei dem das Verhältnis der Querschnittsfläche des Rohrreaktors, die von der inneren Wand des doppelwandigen Leitrohrs begrenzt wird, zu der Querschnittsfläche des Rohrreaktors, die von der Wand des Rohrreaktors und der äußeren Wand des doppelwandigen Leitrohrs begrenzt wird, 1:1 bis 1:3 beträgt.

## Claims

1. Process for the preparation of diisocyanates and triisocyanates of the general formula (I)
R(NCO)ₙ (I),
in which
R represents a (cyclo)aliphatic or aromatic hydrocarbon radical having up to 15 carbon atoms, preferably 4 to 13 carbon atoms, with the proviso that at least 2 carbon atoms are arranged between two NCO groups, and
n represents the number 2 or 3,
by phosgenation of the corresponding diamines and/or triamines of the general formula (II)
R(NH₂)ₙ (II),
in which
R represents a (cyclo)aliphatic or aromatic hydrocarbon radical having up to 15, preferably 4 to 13, carbon atoms, with the proviso that at least two carbon atoms are arranged between two amino groups, and
n represents the number 2 or 3,
in the gas phase in a tubular reactor which has a double-walled guide tube extending centrally in the direction of the axis of rotation of the tubular reactor, a concentric annular gap being formed between the inner and the outer wall of the double-walled guide tube, and the ratio of the cross-sectional area of the tubular reactor, which area is bounded by the inner wall of the double-walled guide tube, to the cross-sectional area of the tubular reactor, which area is bounded by the wall of the tubular reactor and the outer wall of the double-walled guide tube, being 1:0.5 to 1:4, in which the diamines and/or triamines in vapour form and phosgene are heated separately from one another to temperatures of 200°C to 600°C,
and the diamines and/or triamines in vapour form are fed to the tubular reactor via the concentric annular gap at a mean flow velocity of 20-150 m/s and phosgene is fed to the tubular reactor over the remaining cross-sectional areas of the tubular reactor at a mean flow velocity of at least 1 m/s.

2. Process according to Claim 1, in which the mean flow velocity of the diamines and/or triamines in vapour form is 40 to 100 m/s.

3. Process according to either of Claims 1 or 2, in which the mean flow velocity of the phosgene is 5 to 15 m/s.

4. Process according to any of Claims 1 to 3, in which isophoronediamine, hexamethylenediamine or bis(p-aminocyclohexyl)methane are used as diamines.

5. Process according to any of Claims 1 to 3, in which 1,8-diamino-4-(aminomethyl)octane or triaminononane are used as triamines.

6. Process according to any of Claims 1 to 4, in which the phosgenation is carried out in a tubular reactor in which the ratio of the cross-sectional area of the tubular reactor, which area is bounded by the inner wall of the double-walled guide tube, to the cross-sectional area of the tubular reactor, which area is bounded by the wall of the tubular reactor and the outer wall of the double-walled guide tube, is 1:1 to 1:3

## Revendications

1. Procédé de préparation de diisocyanates et de triisocyanates de la formule générale (I) :
R(NCO)ₙ (I)
dans laquelle
R représente un reste hydrocarbure (cyclo)aliphatique ou aromatique ayant jusqu'à 15 atomes de carbone, de préférence 4 à 13 atomes de carbone, avec la condition qu'entre deux groupes NCO, au moins deux atomes de carbone sont présents, et
n représente le nombre 2 ou 3,
par phosgénation de la diamine et/ou triamine correspondante de la formule générale (II) :
R(NH₂)ₙ (II)
dans laquelle
R représente un reste hydrocarbure (cyclo)aliphatique ou aromatique ayant jusqu'à 15 atomes de carbone, de préférence 4 à 13 atomes de carbone, avec la condition qu'entre deux groupes amino, au moins deux atomes de carbone sont présents, et
n représente le nombre 2 ou 3,
en phase gazeuse dans un réacteur tubulaire, qui présente un tube directeur à double paroi centrique, s'étendant en direction de l'axe de rotation du réacteur tubulaire, où entre les parois interne et externe du tube directeur à double paroi, un passage annulaire concentrique est formé, et où le rapport de la coupe transversale du réacteur tubulaire, qui est limité par la paroi interne du tube directeur à double paroi, à la coupe transversale du réacteur tubulaire, qui est limité par la paroi du réacteur tubulaire et la paroi externe du tube directeur à double paroi, se situe dans l'intervalle allant de 1:0,5 à 1:4,
dans lequel la diamine et/ou la triamine et le phosgène sont chauffés séparément l'un de l'autre, à des températures allant de 200°C à 600°C,
la diamine et/ou triamine à l'état de vapeur sont conduites dans le réacteur tubulaire par le passage annulaire concentrique à une vitesse moyenne d'écoulement de 20-150 m/seconde, et le phosgène est conduit dans le réacteur tubulaire par la coupe transversale restante du réacteur tubulaire, à une vitesse moyenne d'écoulement d'au moins 1 m/seconde.

2. Procédé selon la revendication 1, dans lequel la vitesse moyenne d'écoulement de la diamine et/ou triamine à l'état de vapeur se situe dans l'intervalle allant de 40 à 100 m/seconde.

3. Procédé selon la revendication 1 ou 2, dans lequel la vitesse moyenne d'écoulement du phosgène se situe dans l'intervalle allant de 5 à 15 m/seconde.

4. Procédé selon l'une des revendications 1 à 3, dans lequel on met en oeuvre comme diamine, l'isophoronediamine, l'hexaméthylènediamine ou le bis(p-aminocyclohexyl)méthane.

5. Procédé selon l'une des revendications 1 à 3, dans lequel on met en oeuvre comme triamine, le 1,8-diamino-4-(aminométhyl)octane ou triaminononane.

6. Procédé selon l'une des revendications 1 à 4, dans lequel la phosgénation est réalisée dans un réacteur tubulaire, où la coupe transversale du réacteur tubulaire, qui est limité par la paroi interne du tube directeur à double paroi, à la coupe transversale du réacteur tubulaire, qui est limité par la paroi du réacteur tubulaire et la paroi externe du tube directeur à double paroi, se situe dans l'intervalle allant de 1:1 à 1:3.
